# EUROPEAN PATENT SPECIFICATION

(11) **EP 0 253 774 B1**
(45) Date of publication and mention of the grant of the patent: **07.01.1993**
(21) Application number: 87830167.0
(22) Date of filing: 06.05.1987
(51) Int. Cl.: C12M 1/16, C12M 3/04, C12M 1/14

(54) **A process for biological reactions using support-immobilized cells and an apparatus for carrying out the same**
Verfahren für biologische Reaktionen mittels an Trägern immobilisierten Zellen und Vorrichtung zur Durchführung des Verfahrens
Procédé de réactions biologiques avec utilisation de cellules immobilisées sur support et appareil pour effectuer le procédé

(30) Priority: 07.05.1986 IT 4799486
(43) Date of publication of application: 20.01.1988
(73) Proprietor: Ente per le nuove tecnologie, l'energia e l'ambiente (ENEA), I-00198 Roma (IT)
(72) Inventor: Converti, Attilio, I-16132 Genova GE (IT); Del Borghi, Marco, I-16125 Genova GE (IT); Parisi, Federico, I-20137 Milano MI (IT); Ferraiolo, Giuseppe, I-16131 Genova GE (IT)
(74) Representative: Tonon, Gilberto

(56) References cited:
- BE-A- 901 711
- US-A- 3 027 305
- US-A- 4 208 483
- US-A- 4 224 413
- US-A- 4 554 075
- CHEMICAL ABSTRACTS, vol. 83, no. 15, 13 October 1975, page 376, abstract no. 130029r, Columbus, Ohio, US; & JP-A-75 09 868 (YAMASA SHOYU CO., LTD) 16-04-1975

## Description

The present invention relates to a process for carrying out alcoholic fermentation using yeast cells immobilized on spongy supports, consisting of spongy cloth.

Processes are currently known which use cells immobilized on supports which are a striking step forward when compared to continuous processes without supports, in that aid processes show a substantial increase in volumetric productivity due to the increased cell concentration per unit volume.

The support on which the cells are immobilized in fact prevents the cells from washing out of the reactor, even at higher flow rates than those under which "wash-out" occurs for the same reactor with no support. Supports most commonly used up to now for cellular immobilization in continuous processes are rigid synthetic or natural polymeric materials which allow efficient physical entrapment of the cells. The choice of the support is directly related to the operating conditions and to the biological process in question. Materials which have been used for these applications include the following:
a) calcium alginate matrices
b) polyacrylamide gel,
c) k-carragenate matrices,
d) glass fibers,
e) water insoluble ion exchange materials,
f) matrices in which the cell is chemically bound to the support with bifunctional reagents like glutaraldehyde,
g) cellulose tetraacetate fibers.

Even though processes using these supports lead to very high productivity with respect to processes without supports, they also suffer from disadvantages in that they are extremely difficult to regenerate and so reuse. For example, in the case of processes using alginate supports, regeneration is extremely difficult and the process using said support would be optimal if the continuous process were worked indefinitely. Processes which are readily subject to bacterial contamination or to culture aging require periodic renewal through regeneration or replacement of the matrix and new inoculation, leading obviously to increased costs.

Other processes currently used involve immobilization of cells with chemical reagents. This immobilization eliminates any possibility of renewal of the culture due to the strong chemical bonds between the support and cell walls.

In the case of fermentation processes in which gaseous products or by-products are generated (CO₂ in the case of alcoholic fermentation and the aerobic degradation of sewage and CH₄ in the case of anaerobic degradation), other disadvantages are related to the inevitable rupture of the rigid bed by the product gases, with consequent increased loss of charge in the reactor and obstruction in the case of fermentation columns.

### Object of the Invention

It has surprisingly been discovered that a process in which spongy materials consisting of spongy cloth are used as supports for cell immobilization overcomes the disadvantages of the processes using the preceding technique, particularly in the case of alcoholic fermentation.

In order to overcome these disadvantages, the process according to the present invention uses, as supports for cellular entrapment, spongy materials consisting of spongy cloth which offer the following advantages:
a) simple installation of the support in the reactor;
b) simple regeneration of the support by means of washing, thanks to the flexibility of the material;
c) reuse of the support in successive fermentation cycles, with consequent economic advantages;
d) renewal of the cell colonies in the innermost cavities by means of periodic washing operations and inoculation with new cell charges;
e) prevention of contamination due to renewal of the culture;
f) no breakdown of the bed due to the formation of gaseous products or by-products; this resistance to breakdown, particularly important in the case of support filled columns, is guaranteed by the high, uniform prorosity and flexibility of the spongy material;
g) low cost of the spongy cloth materials with respect to more complex synthetic materials;
j) possibility of using spongy materials in various types of continuous reactors, on the basis of the shape and dimensions of the spongy particles.

Therefore the object of the present invention is a process for carrying out alcoholic fermentation using yeast cells immobilized on spongy supports consisting of spongy cloth. This material comprises viscose, cotton and vegetal cellulose in suitable proportions and shows the following porperties:
- absorption of H₂ from 2 to 4 times its weight;
- maximum cellular retention between 1 and 1.5 g of dry substance per gram of support;
- specific area between 28 and 35 cm²/g;
- volumetric variation between 32 and 37%;
- atoxicity;
- sterilizability;
- biodegradability;
- regenerability.

With regard to biodegradability, this term is intended to refer to compounds which return to natural conditions on non-biological treatments. For cellular supports, biodegradability is a negative property because it tends to consume the spongy material. Therefore processes which use highly biodegrading microorganisms require supports which are completely synthetic (polyamides, polyesters, etc.), with no cellulose. In other cases (such as alcoholic fermentation), biodegradability is not a problem and therefore vegetal cellulose may be present in the spongy material. With respect to alginates, carragenates, etc., used in processes belonging to the state of the art, the spongy cloth support is advantageous not only for economic reasons, since it is much less expensive than the other two above referred supports, but also because it allows more practical solutions at the industrial level. In fact with the substances cited above and in a continuous process, no matter what geometric shape the substance assumes, it must be replaced often, stopping the process. Furthermore, the above substances (alginate, carragenate) are not sterilizable, are often toxic and may undergo chemical changes during the process.

The process according to the present invention relates to the transformation of hydrolised starch and/or molasses into ethanol by yeast cells making use of the spongy cloth with the properties and chemical composition above indicated.

The word molasses refers to a by product of the sugar processing industry.

The process according to the present invention is of particular interest when applied to particular types of reactors used in techniques belonging to the state of the art, especially in the case of so-called biodisk reactors (RBS).

### Brief description of the drawing

Figure 1 is a cross-section view of a biodisk reactor with spondy material applied to the disks.

Figure 2 is a flow diagram of a continuous process which uses the biodisk reactor with the spongy material attached to the disks.

Figure 3 is a graph comparing a one and/or two step biodisk reactor with the spongy cloth support with a one and/or two step biodisk reactor without the spongy cloth support, in the case of alcoholic fermentation.

An example of the spongy material particularly suitable in the process according to the invention is the sponge cloth "WETTEX"®, a registered trademark which typifies a range of products used in the cleaning field, even though there are other analogous products commercially available which show the properties and chemical composition above indicated. The use of this spongy support in the process according to the present invention makes it possible the achievement of the advantages cited above with respect to other supports belonging to the state of the art.

An apparatus comprising a rotating disk biological reactor with spongy cloth support on the disks is described with reference to the attached drawings.

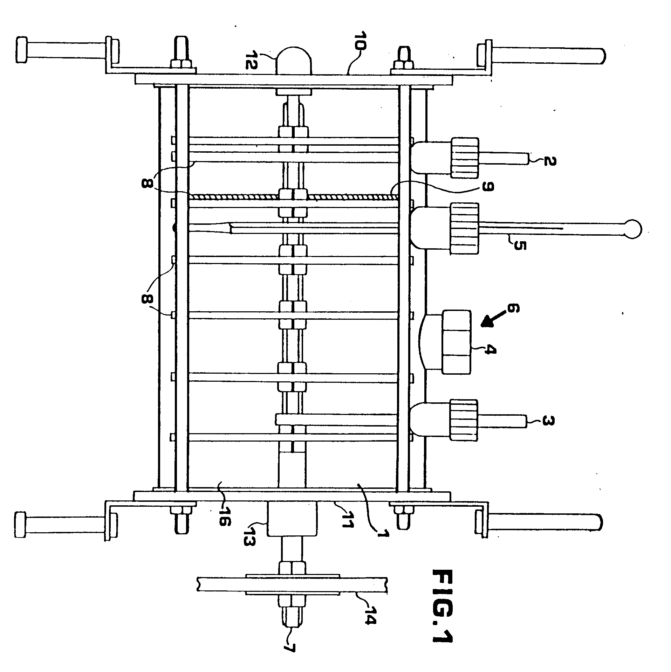

In figure 1, said apparatus comprises a reactor immersed in a thermostatic bath consisting of a fluid (for example water) in a steel container (not shown) equipped with an electrical heating system controlled by a suitably calibrated thermostat, so as to maintain the desired temperature in the reactor. In the process described below as an example, said temperature is 30°C.

Said container contains one or more reactors, since the reaction may be modular, each comprising a hollow cylinder (1) of PYREX® glass closed at the ends with two steel flanges (10) and (11) and arranged horizontally. The top part of said cylinder contains some openings for the input (2) and outlet (3) of the nutrient medium as well as for sample taking (4), temperature measurement (5) and pH adjustment (6). Said cylinder (1) contains a central horizontal shaft (7) with steel disks (8) of suitable diameter (slightly less than the diameter of the cylinder) on which is placed the spongy material (9) where the cell entrapment will occur. Said disks are immersed in nutrient medium for about half of their circumference and also integral with the shaft (7). One end of said shaft rotates in a blind cavity (12) in the centre of the flange (10), while the other end is integral with a driven pulley (14) through a stuffing box (13) coupled to the second flange (11). Said pulley is in turn connected by a belt to a drive pulley integral with the shaft of a variable speed motor (said belt-pulley-motor complex is not shown in the figure).

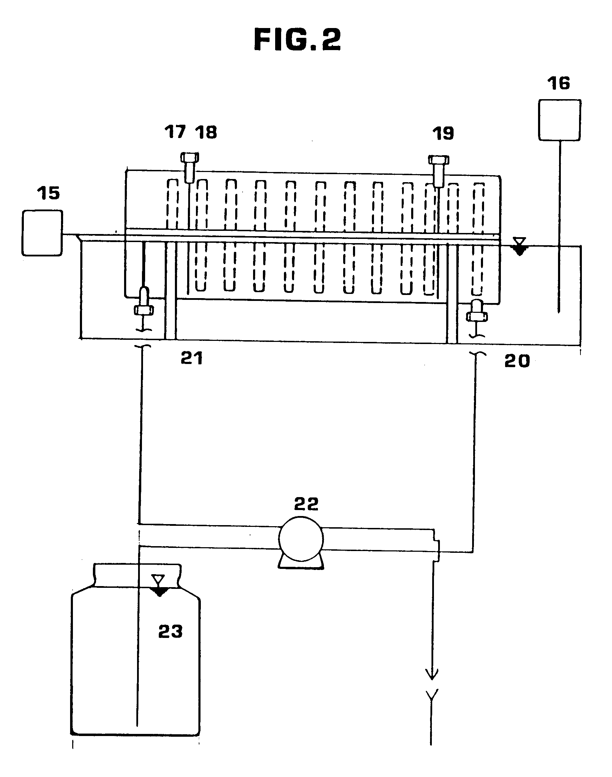

Figure 2 is a schematic diagram of the system showing the motor for operating the axle (15), the control and automatic adjustment of the temperature (16) and pH (17-18), the thermometer (19), the inlet (20) and outlet (21) for the fermented "must".

A peristaltic pump is shown (22) which feeds nutrient medium into the reactor in a continuous flow. Also shown is the feed vessel (23).

An example of the process according to the present invention making use of the above described apparatus is provided in the following.

A sugar base substrate is used (glucose and/or saccharose obtained from hydrolised starch and/or molasses) with a sugar content of approximately 50%. Said substance is taken from a suitable sterilized vessel, after treatment with 2 g/liter of (NH₄)₂SO₄ (ammonium sulfate), 0.4 g/liter of MgSO₄.7H₂O, 5 g/liter of KH₂PO₄ (potassium biacidphosphate). Said substance is then treated with a 0.03 molar sodium citrate buffer solution (1 molar citric acid with dissolved NaOH solution) until a pH of 5 is reached (the optimal pH for fermentation). Before being placed in the reactor, said substance is brought up to volume by dilution to the desired concentration and sterilized at 116°C for 20 minutes. Following this preparation, the reactor is charged continuously at optimal flow with the nutrient substance by means of an adjustable peristaltic pump. The optimal fermentation pH is continuously adjusted (to a value of 5) inside the reactor by means of a pH meter connected to a control module which, by means of another peristaltic pump, automatically injects the solution necessary for pH stabilization (2.5N Na₂CO₃).

The fermentation process is begun by injecting a yeast cell suspension into the reactor after input of the nutrient medium. The cellular suspension distributes itself uniformly inside the spongy matrix and begins to reproduce abundantly, forming large colonies in the cavities of the spongy support during the process. The system reaches operating conditions after approximately 10 days. The level of the liquid in the reactor is adjusted and stabilized at approximately half the volume of the reactor, by means of another peristaltic pump or of the previous one with two stages, which peristaltic pump removes an identical volume for each input into the reactor. Flow adjustment requires particulars such as the arrangement of the input tube at the bottom of the reactor and of the oulet tube at the water level, with the latter at a greater flow rate than the former. This is continued until the level of the liquid establishes itself at half the volume of the reactor. With reference to the process described above, the nutrient is transformed inside the reactor into the final product (ethanol) in approximately 3 hours. It should be noted that in reactors with no spongy matrix this tranformation is approximately 4 times as long. Therefore the reactor produces, under optimal condition, an hourly flow approximately equal to a third of the volume of the solution in the reactor (sugar concentration 100 g/liter). By the continuous process therefore the nutrient medium is almost completely replaced in approximately 3 hours, providing an alcohol solution as the final product.

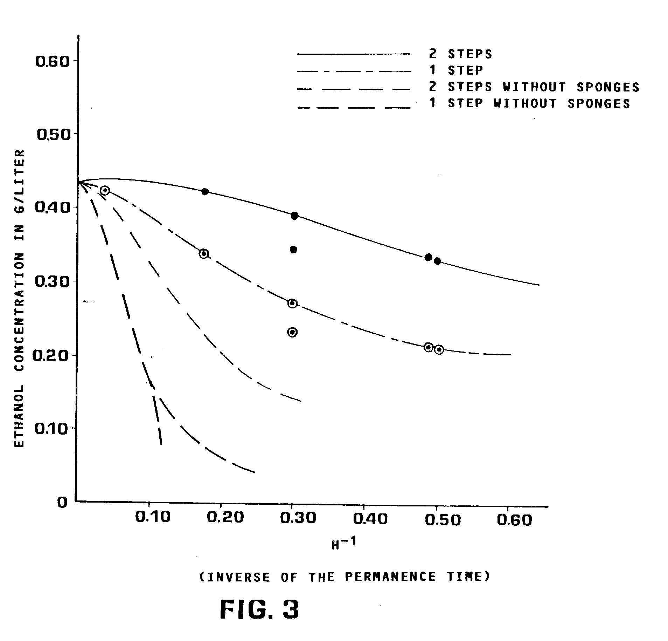

Shown in figure 3 is a graph comparing an ethanol production process with a biodisk reactor with spongy support and without spongy support. The graph clearly shows that ethanol production is much more efficient in the presence of the spongy supports.

## Claims

1. A process for carrying out alcoholic fermentation using yeast cells immobilized on spongy supports starting from hydrolised starch and/or molasses
characterized by the fact that said spongy supports are made of spongy cloth consisting of viscose, cotton and vegetal cellulose and showing the following properties:
atoxicity;
sterilizability;
biodegrability;
regenerability;
absorbtion of water from 2 to 4 times its weight;
maximum cellular retention between 1 and 1.5 g of dry substance/g of support;
specific area between 28 and 35 cm²/g;
volumetric variation between 32 and 37 %.

2. A process according to claim 1, characterized by the fact that said spongy cloth supports are made of Wettex ^{(R)} spongy cloth.

## Patentansprüche

1. Verfahren zum Ausführen von alkoholischer Fermentation unter Verwendung von auf schwammigen Trägern immobilisierten Hefezellen, ausgehend von hydrolysierter Stärke und/oder Melasse,
**dadurch gekennzeichnet,** daß die schwammigen Träger aus schwammigem Stoff gemacht sind, welcher aus Viskose, Baumwolle und pflanzlicher Zellulose besteht und die folgenden Eigenschaften aufweist:
Ungiftigkeit;
Sterilisierbarkeit;
biologische Abbaubarkeit;
Regenerierbarkeit;
Absorption des 2 bis 4-fachen seines Gewichts an Wasser;
maximale Zell-Retention zwischen 1 und 1,5 g Trockensubstanz/ g des Trägers;
spezifische Fläche zwischen 28 und 35 cm²/g;
volumetrische Variation zwischen 32 und 37%.

2. Verfahren nach Anspruch 1, **dadurch gekennzeichnet,** daß die schwammigen Stoffträger aus schwammigen Wettex®-Stoff gemacht sind.

## Revendications

1. Procédé destiné à effectuer la fermentation alcoolique en utilisant des cellules de levure immobilisées sur des supports spongieux en partant d'amidon hydrolysé et/ou mélasses,
caractérisé par le fait que ces supports spongieux sont réalisés à partir d'un tissu de mousse constitué par de la viscose, du coton et de la cellulose végétale et présentant les propriétés suivantes :
atoxicité ;
stérilisabilité ;
biodégradabilité ;
régénérabilité ;
absorption de l'eau à partir de 2 à 4 fois son poids;
rétention cellulaire maximum entre 1 et 1,5 g de matière sèche/g de support ;
surface effective entre 28 et 35 cm²/g ;
variation volumétrique entre 32 et 37 %.

2. Procédé selon la revendication 1, caractérisé par le fait que les supports en tissu éponge sont réalisés à partir de tissu éponge Wettex®.
